# EUROPEAN PATENT APPLICATION

(11) **EP 4 825 318 A1**
(43) Date of publication of application: **21.10.2026**
(21) Application number: 25742275.8
(22) Date of filing: 17.01.2025
(51) Int. Cl.: A61B 5/346, A61B 5/0205, A61B 5/00, G16H 50/20, G16H 50/30, G16H 40/20

(54) **COMPUTING DEVICE FOR SETTING MEDICAL TREATMENT ORDER ON BASIS OF PATIENT?S CONDITION AND METHOD FOR CONTROLLING SAME**

(30) Priority: 17.01.2024 KR 20240007257
(71) Applicant: Medical AI Co., Ltd., Seoul 06180 (KR)
(72) Inventor: KWON, Joonmyoung, Seoul 06180 (KR); CHO, Hyunji, Seoul 06180 (KR)
(74) Representative: Patentanwaltskanzlei Matschnig & Forsthuber OG
(86) International application number: PCT/KR2025/000999
(87) International publication number: WO 2025/155127

(57) **Abstract**

The present disclosure provides a method and a computing device for setting a treatment order based on an emergency patient's condition. According to an embodiment of the present disclosure, a method for setting a treatment order based on an emergency patient's condition, performed by a computing device including at least one processor, comprises, when reception data of a new examinee is acquired, acquiring physiological data of the examinee via a sensing unit, analyzing the physiological data using a pre-trained neural network model to identify the new examinee's condition, and setting a treatment order for the new examinee based on a time at which the reception data of the new examinee is acquired and the new examinee's condition. The setting of the treatment order comprises setting the treatment order for the new examinee by adjusting, based on the examinee's condition, the treatment orders of the other previously registered examinees who are waiting for treatment.

## Description

### TECHNICAL FIELD

The present disclosure relates to a computing device for setting a treatment order based on a patient's condition and a control method thereof, and more particularly, to a device and a control method thereof for analyzing physiological data acquired from a patient to identify the patient's condition, and adjusting treatment orders of a plurality of registered patients.

### BACKGROUND OF THE INVENTION

Currently, in most hospital emergency rooms, a treatment order is generally determined according to the order in which patients are registered. Although medical staff classify the severity of a patient's condition through an initial assessment, a system for continuously monitoring changes in the patient's condition thereafter or adjusting the treatment order in real time based on this is insufficient. This systemic limitation may pose a greater risk to a patient who does not have a guardian. In particular, cases have recently been reported where a patient, who was classified as having a mild condition and had been waiting for a long time, died because a sudden deterioration in his or her condition was not detected in a timely manner.

Such a problem arises from structural limitations occurring in an emergency room environment and the heavy workload of medical staff. In a situation of managing a large number of patients, medical staff are in a difficult position to periodically check the condition changes of all patients, and in the case of a patient without a guardian, there is a high possibility that the medical staff may not recognize signs of a deteriorating condition. This limitation not only degrades the efficiency of emergency room operations but also has a serious impact on patient safety. To date, technical approaches have focused on evaluating a patient's initial condition or on reception management, and a sufficient solution that can detect changes in a patient's condition in real time and adjust the treatment order or respond appropriately based thereon has not been provided.

### SUMMARY OF THE INVENTION

### TECHNICAL PROBLEM

The present disclosure is contrived in response to the aforementioned background, and an object thereof is to provide a computing device and a control method thereof for setting a treatment order based on an emergency patient's condition and adjusting a preset treatment order.

However, the problems to be solved by the present disclosure are not limited to the aforementioned problems, and other unmentioned problems will be clearly understood from the following description.

### TECHNICAL SOLUTION

To achieve the aforementioned objects, a method for setting a treatment order based on a patient's condition, performed by a computing device including at least one processor, comprises, when reception data of a new examinee is acquired, acquiring physiological data of the examinee via a sensing unit, analyzing the physiological data using a pre-trained neural network model to identify the new examinee's condition, setting a treatment order for the new examinee based on a time at which the reception data of the new examinee is acquired and the new examinee's condition, and providing queue number information corresponding to the treatment order of the new examinee. The setting of the treatment order comprises setting the treatment order for the new examinee by adjusting, based on the examinee's condition, the treatment orders of the other previously registered examinees who are waiting for treatment.

Alternatively, the setting of the treatment order for the new examinee comprises setting the treatment order for the new examinee to be later than the treatment orders of the other examinees waiting for treatment, based on a reception time at which the reception data of the new examinee is acquired.

Alternatively, the setting of the treatment order for the new examinee by adjusting, based on the new examinee's condition, the treatment orders of the other previously registered examinees who are waiting for treatment comprises, when it is identified, based on the new examinee's condition, that the new examinee's risk level is higher than the risk levels of the other examinees waiting for treatment, setting the treatment order for the new examinee to be earlier than the treatment orders of the other examinees waiting for treatment.

Alternatively, the new examinee's risk level is classified into a plurality of condition grades, and the setting of the treatment order for the new examinee to be earlier than the treatment orders of the other examinees waiting for treatment comprises, when there are a plurality of other examinees waiting for treatment, identifying, from among the plurality of other examinees, an examinee having a condition grade lower than the condition grade of the new examinee's risk level, and setting the treatment order for the new examinee to be earlier than the treatment order of the identified examinee.

Alternatively, the new examinee's condition is classified into a plurality of condition grades, and a re-measurement period for the physiological data is set corresponding to each of the condition grades. The method comprises identifying a condition grade corresponding to the new examinee's condition, and providing re-measurement time information for the physiological data according to the re-measurement period corresponding to the new examinee's condition grade.

Alternatively, when the re-measurement time is reached, the method comprises displaying information requesting the re-measurement of the physiological data of the new examinee via a display of the computing device, or transmitting information requesting the re-measurement of the physiological data to a terminal device of the new examinee via a communication interface.

Alternatively, when it is identified that, after displaying or transmitting the information requesting the re-measurement, the new examinee has not re-measured the physiological data for a preset time, the method comprises transmitting information on the new examinee to a terminal device of an administrator.

Alternatively, the method comprises re-identifying the examinee's condition based on the physiological data re-acquired from the new examinee via the sensing unit upon re-measurement by the new examinee, and adjusting the treatment order of the examinee based on the examinee's re-identified condition.

Alternatively, queue numbers of the other examinees waiting for treatment are set and issued at a preset interval corresponding to the treatment orders of the other examinees waiting for treatment. When the treatment order for the new examinee is set to be earlier than the treatment orders of the other examinees waiting for treatment, the method comprises maintaining the queue numbers of the other examinees whose treatment orders have been adjusted to be later, and setting and issuing, for the new examinee, a queue number that falls within the preset interval and is earlier than the queue numbers of the other examinees whose treatment orders have been adjusted to be later.

Alternatively, the condition of the other examinees waiting for treatment is classified into a plurality of condition grades, and the preset interval is set to correspond to the condition grade of the other examinees waiting for treatment.

To achieve the aforementioned objects, a computing device for setting a treatment order based on a patient's condition, may comprise a memory storing program code, a sensing unit configured to acquire physiological data of an examinee, and one or more processors configured to, when reception data of a new examinee is acquired, acquire physiological data of the examinee via the sensing unit, analyze the physiological data using a pre-trained neural network model to identify the new examinee's condition, set a treatment order for the new examinee based on a time at which the reception data of the new examinee is acquired and the new examinee's condition, and provide queue number information corresponding to the treatment order of the new examinee. The one or more processors may be configured to set the treatment order for the new examinee by adjusting, based on the examinee's condition, the treatment orders of the other previously registered examinees who are waiting for treatment.

To achieve the aforementioned objects, a computer program stored on a computer-readable storage medium, the computer program, when executed by one or more processors, causes operations for setting a treatment order based on a patient's condition to be performed. The operations comprise, when reception data of a new examinee is acquired, acquiring physiological data of the examinee via a sensing unit, analyzing the physiological data using a pre-trained neural network model to identify the new examinee's condition, setting a treatment order for the new examinee based on a time at which the reception data of the new examinee is acquired and the new examinee's condition, and providing queue number information corresponding to the treatment order of the new examinee. The setting of the treatment order comprises setting the treatment order for the new examinee by adjusting, based on the examinee's condition, the treatment orders of the other previously registered examinees who are waiting for treatment.

### ADVANTAGEOUS EFFECTS

According to a computing device for setting a treatment order based on a patient's condition according to an embodiment of the present disclosure, a change in condition may be automatically detected by monitoring the patient's physiological data in real time. As a result, an emergency patient may be quickly identified, and a treatment order may be dynamically adjusted to induce a response from medical staff in a timely manner. Consequently, it is possible to prevent a deterioration of the patient's condition and contribute to the efficient use of medical resources and an improvement in the patient's survival rate.

### BRIEF DESCRIPTION OF THE DRAWINGS

FIG. 1 is a diagram illustrating a computing device for setting a treatment order based on a patient's condition according to an embodiment of the present disclosure.
FIG. 2 is a schematic block diagram of a computing device 100 according to an embodiment of the present disclosure.
FIG. 3 is a flowchart schematically illustrating a method for setting a treatment order based on a patient's condition according to an embodiment of the present disclosure.
FIG. 4A and FIG. 4B are diagrams illustrating a method for setting a treatment order based on a patient's condition according to an embodiment of the present disclosure.
FIG. 5 is another diagram illustrating a method for setting a treatment order based on a patient's condition according to an embodiment of the present disclosure.
FIG. 6 is another diagram illustrating a method for setting a re-measurement period according to a patient's condition according to an embodiment of the present disclosure.
FIG. 7 is a diagram illustrating the issuance of queue numbers for examinees at a preset interval according to an embodiment of the present disclosure.
FIG. 8 is a diagram illustrating the issuance of a queue number for an examinee at an interval set according to a condition grade of the examinee according to an embodiment of the present disclosure.
FIG. 9 is a configuration diagram of a computing device according to another embodiment of the present disclosure.

### DETAILED DESCRIPTION OF THE EMBODIMENTS

Hereinafter, the present disclosure describes embodiments in detail with reference to the accompanying drawings so that a person of ordinary skill in the art to which the present disclosure pertains can easily implement the same. The embodiments presented in the present disclosure are provided to enable a person of ordinary skill in the art to use or implement the content of the present disclosure. Accordingly, various modifications to the embodiments of the present disclosure will be apparent to a person of ordinary skill in the art. That is, the present disclosure may be embodied in many different forms and is not to be construed as limited to the embodiments set forth herein.

Throughout the specification of the present disclosure, the same or similar reference numerals refer to the same or similar components. Furthermore, for clarity of description of the present disclosure, reference numerals for parts unrelated to the description of the present disclosure in the drawings may be omitted.

The term "or" as used in the present disclosure is intended to mean an inclusive "or" rather than an exclusive "or". That is, unless otherwise specified or clear from the context, "X uses A or B" is to be understood to mean any of the natural inclusive permutations. For example, if "X uses A or B" is used in the present disclosure, unless otherwise specified or clear from the context, it may be interpreted as any of the cases where X uses A, X uses B, or X uses both A and B.

The term "and/or" as used in the present disclosure is to be understood to refer to and include all possible combinations of one or more of the associated listed concepts.

The terms "comprise" and/or "comprising" as used in the present disclosure are to be understood to mean the presence of a specific feature and/or component. However, the terms "comprise" and/or "comprising" are to be understood as not precluding the presence or addition of one or more other features, other components, and/or combinations thereof.

In the present disclosure, unless otherwise specified or clear from the context to indicate a singular form, generally the singular is to be interpreted to include "one or more".

The term "N-th (where N is a natural number)" as used in the present disclosure may be understood as an expression used to distinguish components of the present disclosure from each other according to a certain standard, such as a functional perspective, a structural perspective, or for convenience of explanation. For example, in the present disclosure, components that perform different functional roles may be distinguished as a first component or a second component. However, components that are substantially the same within the technical spirit of the present disclosure but need to be distinguished for convenience of explanation may also be distinguished as a first component or a second component.

The term "acquire" as used in the present disclosure may be understood as not only receiving data via a wired/wireless communication network from an external device or system, but also generating data in an on-device form.

Meanwhile, the term "module" or "unit" as used in the present disclosure may be understood as a term referring to an independent functional unit that processes computing resources, such as a computer-related entity, firmware, software, or a portion thereof, hardware or a portion thereof, or a combination of software and hardware. A "module" or a "unit" may be a unit configured as a single element, or a unit expressed as a combination or a set of a plurality of elements. For example, in a narrow sense, a "module" or a "unit" may refer to a hardware element of a computing device or a set thereof, an application program that performs a specific function of software, a processing procedure implemented by software execution, or a set of instructions for program execution. Furthermore, in a broad sense, a "module" or a "unit" may refer to a computing device itself that constitutes a system, or an application executed on the computing device. However, the above-mentioned concepts are only examples, and the concepts of "module" or "unit" may be variously defined within the scope understandable by a person of ordinary skill in the art based on the content of the present disclosure.

The term "model" as used in the present disclosure may be understood as an abstraction for a system implemented using mathematical concepts and language to solve a specific problem, a set of software units for solving a specific problem, or a processing procedure for solving a specific problem. For example, a neural network "model" may refer to an entire system implemented as a neural network that has a problem-solving ability through learning. The neural network may have problem-solving ability by optimizing parameters that connect nodes or neurons through learning. A neural network "model" may include a single neural network or a set of neural networks in which a plurality of neural networks are combined.

The term "data" as used in the present disclosure may include "images", signals, and the like. The term "image" as used in the present disclosure may refer to multi-dimensional data composed of discrete image elements. In other words, an "image" may be understood as a term referring to a digital representation of an object that can be seen by the human eye. For example, an "image" may refer to multi-dimensional data composed of elements corresponding to pixels in a 2D image. An "image" may refer to multi-dimensional data composed of elements corresponding to voxels in a 3D image.

The foregoing description of the terms is for the purpose of aiding the understanding of the present disclosure. Therefore, it is to be noted that if the foregoing terms are not explicitly stated as limiting the content of the present disclosure, they are not used in a sense that limits the technical spirit of the content of the present disclosure.

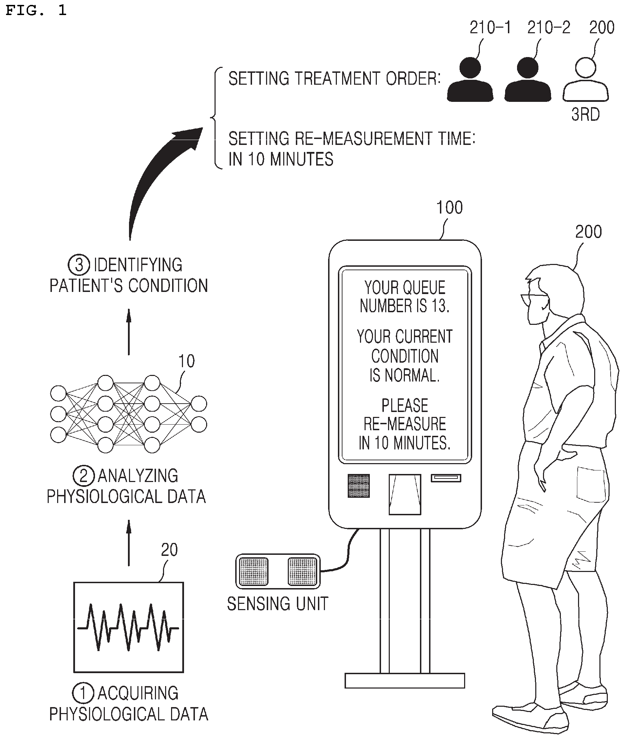

FIG. 1 is a diagram illustrating a computing device for setting a treatment order based on a patient's condition according to an embodiment of the present disclosure.

A computing device 100 according to an embodiment of the present disclosure may be a hardware device or a part of a hardware device that performs comprehensive processing and computation of data, or may be a software-based computing environment connected via a communication interface. For example, the computing device 100 may be a server that is an entity that performs intensive data processing functions and shares resources, or may be a client that shares resources through interaction with a server. In addition, the computing device 100 may be a cloud system or a server in which a plurality of servers and clients interact to comprehensively process data. The above description is merely an example related to the type of the computing device 100, and the type of the computing device 100 may be variously configured within the scope understandable by a person of ordinary skill in the art based on the content of the present disclosure. For example, the computing device 100 may be implemented as a device that identifies and manages a patient who has visited a medical facility (e.g., a hospital, a public health center, a regional emergency medical center). To this end, the computing device 100 may be implemented as various electronic devices installed in a medical facility, such as a desktop, a laptop, a smartphone, a server device, a smart band, a smart watch, a smart ring, a kiosk device, or a biosignal measurement device. For example, the computing device 100 may be implemented as a kiosk device installed in a medical facility to register patients, or may be implemented as a public smart watch (or a biosignal measurement device) installed in a medical facility to acquire a patient's physiological data. However, hereinafter, for the understanding of the present disclosure, the computing device 100 is described assuming it is a kiosk device.

According to an embodiment of the present disclosure, the computing device 100 may perform a function of managing a patient who has visited a medical facility where the computing device 100 is installed. Specifically, the computing device 100 may acquire reception data regarding a patient who has visited a medical facility. The reception data may include the purpose of the patient's visit to the medical facility, the patient's personal information (name, gender, resident registration number, and the like), and the patient's biological information (height, weight, and the like). The computing device 100 may determine the patient's queue position by identifying the number of the other waiting patients related to the patient's visit purpose, such as treatment, examination, or payment, based on the reception data, and may generate and output a queue number corresponding to the queue position.

In particular, referring to FIG. 1, a computing device 100 according to an embodiment of the present disclosure may include a sensing unit 130 to directly acquire physiological data 20 of a patient, particularly a patient who has visited for the purpose of treatment (an examinee). The physiological data 20 may include various data measured and acquired from a patient, such as the patient's body temperature, blood pressure, heart rate, electrocardiogram (ECG), electroencephalogram (EEG), and the like, and the sensing unit 130 may include a sensing module for acquiring each piece of physiological data 20. For example, the computing device 100 may measure a patient's ECG using at least one electrode included in the sensing unit 130 to acquire the patient's ECG data.

The computing device 100 may analyze the acquired ECG data to identify the patient's condition. In particular, the computing device 100 may analyze the ECG data using a pre-trained neural network model 10 to identify the patient's condition. The patient's condition may be a health condition such as the possibility of developing a specific disease, and the risk of a disease. For example, the specific disease may be arrhythmia, left ventricular systolic dysfunction, or the like.

Referring to FIG. 1, according to an embodiment of the present disclosure, the computing device 100 may determine the orders of patients according to their identified conditions. In particular, the computing device 100 may determine the orders of patients such that the more urgent the patient's condition, the sooner the patient receives treatment (or examination) than other waiting patients. For example, if the computing device 100 determines that there is a high probability of developing a specific disease as a result of analyzing the ECG data, it may determine that the patient's condition is urgent. When the computing device 100 classifies the patient's condition into a plurality of condition grades according to the degree of possibility of developing a disease, when the condition grade corresponding to the patient's condition corresponds to a preset condition grade with a high degree of possibility among the plurality of condition grades, it may determine that the patient's condition is urgent. Accordingly, the treatment orders of the other waiting patients may be adjusted. This is to identify an urgent patient who needs urgent treatment that is difficult to detect because it is not visible to the naked eye, and to prevent the death of an urgent patient that may occur while waiting for treatment by adjusting the treatment order so that the patient can receive prompt treatment and care.

In addition, according to an embodiment of the present disclosure, the computing device 100 may set a re-measurement period for the patient according to the identified condition, and may request the patient to perform a re-measurement to acquire physiological data 20 at each set re-measurement period. For example, the computing device 100 may request a re-measurement from the patient's terminal device (e.g., the patient's smartphone, smart watch) based on the terminal device information of the patient included in the reception data, or may request a re-measurement through a speaker of the computing device 100 or a speaker installed in the medical facility.

This is to identify changes in a patient's condition in real time by monitoring an urgent patient waiting in a medical facility without the intervention of medical staff. In addition, if the condition of an urgent patient deteriorates or there is no response from the urgent patient, the computing device can protect the life of the urgent patient by promptly deploying medical staff, minimize the risk due to the deterioration of the condition, and enable effective treatment intervention.

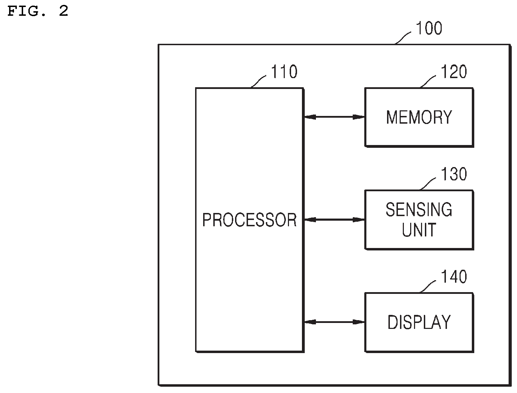

Hereinafter, embodiments of the present disclosure related thereto are described in detail with reference to FIGS. 2 to 5.

FIG. 2 is a schematic block diagram of a computing device 100 according to an embodiment of the present disclosure.

Referring to FIG. 2, a computing device 100 according to an embodiment of the present disclosure may include one or more processors 110 (hereinafter, a processor 110), a memory 120, a sensing unit 130, and a display 140. However, FIG. 1 is only an example, and thus the computing device 100 may further include other components for implementing a computing environment. In addition, only some of the disclosed components may be included in the computing device 100.

The processor 110 according to an embodiment of the present disclosure may be understood as a component unit including hardware and/or software for performing computing operations. For example, the processor 110 may execute a computer program and perform data processing for machine learning. The processor 110 may process computational operations such as feature extraction for machine learning and error calculation based on backpropagation. The processor 110 for performing such data processing may include a central processing unit (CPU), a general-purpose graphics processing unit (GPGPU), a tensor processing unit (TPU), an application-specific integrated circuit (ASIC), or a field-programmable gate array (FPGA). The types of the processor 110 described above are only examples, and the type of the processor 110 may be variously configured within the scope understandable by a person of ordinary skill in the art based on the content of the present disclosure.

The processor 110 is electrically connected to other constituent elements (i.e., the memory 120, the sensing unit 130, and the display 140) of the computing device 100 to control the overall operation of the computing device 100.

A memory 120 according to an embodiment of the present disclosure may be understood as a constituent unit including hardware and/or software for storing and managing data processed in a computing device 100. That is, the memory 120 may store any form of data generated or determined by the processor 110 and any form of data received by a communication interface. For example, the memory 120 may include at least one storage medium of a flash memory type 120, a hard disk type, a multimedia card micro type, a card type memory 120, a random access memory (RAM), a static random access memory (SRAM), a read-only memory (ROM), an electrically erasable programmable read-only memory (EEPROM), a programmable read-only memory (PROM), a magnetic memory 120, a magnetic disk, or an optical disk. In addition, the memory 120 may include a database system that controls and manages data in a predetermined system. The foregoing types of the memory 120 are only one example, and the type of the memory 120 may be variously configured within a scope understandable by a person of ordinary skill in the art based on the content of the present disclosure.

The memory 120 may structure, organize, and manage data necessary for the processor 110 to perform operations, combinations thereof, and program code executable by the processor 110. For example, a neural network model 10 trained to predict a heart disease based on physiological data 20 may be stored in the memory 120. In addition, the memory 120 may include program code for performing learning on the acquired neural network model 10 and training data (e.g., a plurality of physiological data 20 and a plurality of heart disease information matching each physiological data 20). In addition, the memory 120 may store program code for causing the neural network model 10 to receive the physiological data 20 and perform inference according to the purpose of the computing device 100, and processed data generated as the program code is executed.

The sensing unit 130 may acquire physiological data 20 for a patient. For example, the sensing unit 130 may include at least one electrode. In this case, the processor 110 may acquire a patient's ECG data via the at least one electrode. In addition, the sensing unit 130 may include an image sensor or an optical sensor, and the processor 110 may acquire physiological data 20 such as a photoplethysmography (PPG) signal via the image sensor (or the optical sensor).

On the other hand, the sensing unit 130 may be implemented as an external biosignal measurement device that can acquire a patient's physiological data 20 and may be connected to the computing device 100 electrically or via a wired/wireless network.

The display 140 may display 140 various images. In this case, an image includes both a still image and a moving image. The display 140 may output a risk level or condition information identified based on the patient's physiological data 20, or may output the patient's queue number. The display 140 may be implemented in various forms such as a Liquid Crystal Display (LCD) Panel, an Organic Light Emitting Diode (OLED), a Liquid Crystal on Silicon (LCoS), or a Digital Light Processing (DLP). In addition, the display 140 may also include a driving circuit, a backlight unit, and the like, which may be implemented in a form such as an a-si TFT, a low temperature poly silicon (LTPS) TFT, or an organic TFT (OTFT).

On the other hand, the display 140 may be combined with a touch panel to be implemented as a touch screen, and in this case, the display 140 may perform not only an output interface function of outputting an image via the touch screen but also an input interface function of receiving a touch input from a patient. The display 140 may acquire a patient's reception data via a touch input.

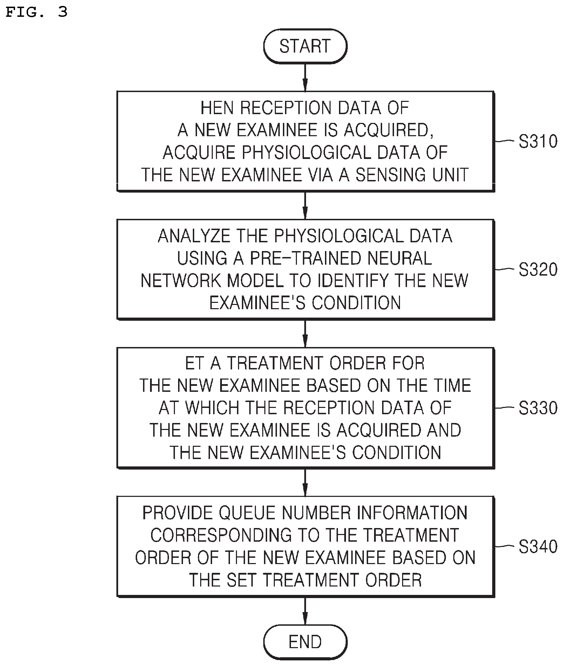

FIG. 3 is a flowchart schematically illustrating a method for setting a treatment order based on a patient's condition according to an embodiment of the present disclosure. Steps S310 to S340 illustrated in FIG. 3 may be further divided into additional steps or combined into fewer steps according to an embodiment of the present disclosure. In addition, some steps may be omitted as necessary, and the order between steps may be changed.

Referring to FIG. 3, when reception data of a new examinee is acquired, the processor 110 may acquire physiological data 20 of the new examinee via the sensing unit 130 (S310). For example, the processor 110 may acquire reception data of a new examinee via the display 140 of the kiosk device. In this case, an examinee is a user who has visited a medical facility for the purpose of treatment among users who have visited the medical facility, and the processor 110 may identify the examinee based on the visit purpose or the like contained in the user's reception data or entered by the user during the process of acquiring the reception data.

When reception data of a new examinee is acquired, the processor 110 may acquire physiological data 20 of the new examinee via the sensing unit 130 of the computing device. To explain again with the above example, the processor 110 may measure a patient's ECG and acquire ECG data about the patient by having at least one electrode included in the sensing unit 130 attached to the user's body. In this case, the processor 110 may acquire a 1-lead ECG for the patient using one electrode, or may acquire various forms of ECG according to 3-lead, 6-lead, and 12-lead methods.

On the other hand, the processor 110 may acquire reception data of a new examinee from an external computing device via a communication interface. For example, the processor 110 may acquire physiological data 20 from a wearable device (e.g., a smart watch) including the sensing unit 130 worn by a new examinee. Alternatively, the processor 110 may acquire physiological data 20 from a server device that communicates in common with the computing device and the wearable device of the new examinee. In this case, the processor 110 may acquire condition information of the new examinee, which is a result of analyzing the physiological data 20, along with the physiological data 20.

When physiological data 20 of a new examinee is acquired, the processor 110 analyzes the physiological data 20 using a pre-trained neural network model 10 to identify the new examinee's condition (S320).

The processor 110 may identify a patient's condition using a pre-trained neural network model 10. The pre-trained neural network model 10 may be a model trained to identify a patient's condition based on training data including input data regarding specific physiological data 20 and label data in which the patient's condition is labeled. For example, when the physiological data 20 is ECG data and the disease type is left ventricular systolic dysfunction, the neural network model 10 may be trained based on training data including input data including a plurality of ECG data and label data in which a label of the presence (or degree) of left ventricular systolic dysfunction is assigned to each input data, and may be trained to minimize the error between the output corresponding to the input data and the label data by a backpropagation algorithm. The neural network model 10 trained in this way may, when ECG data is input, extract feature information (e.g., a feature point) from the ECG data and output a score (or a probability value) regarding the possibility of left ventricular systolic dysfunction of an examinee.

The neural network model 10 may include a plurality of neural network models 10 according to the type of physiological data 20 and the type of the patient's condition to be identified through the physiological data 20 (e.g., a disease type, a type of information to be identified regarding a disease). For example, the neural network model 10 may include a plurality of neural network models 10 that respectively identify whether an examinee has arrhythmia and left ventricular systolic dysfunction, and each neural network model 10 may respectively output a score regarding the possibility of arrhythmia and left ventricular systolic dysfunction of the examinee.

To this end, the neural network model 10 may be implemented as a Multi-Layer Perceptron (MLP), a Convolutional Neural Network (CNN), a Recurrent Neural Network (RNN), a Generative Adversarial Network (GAN), or the like. In this case, the neural network model 10 may include a plurality of residual blocks that extract latent features of the input physiological data 20 and a classifier that classifies a heart disease based on the extracted latent features.

On the other hand, the neural network model 10 may be stored in the memory 120 of the computing device 100, or may be stored in an external computing device connected to the computing device 100. When using the neural network model 10 stored in an external computing device, the computing device 100 transmits the physiological data 20 acquired via a communication interface to the external computing device, and may acquire a result value of the neural network model 10 regarding the patient's condition (i.e., a score indicating the presence or absence of a specific disease or the degree of possibility of a specific disease) from the external computing device.

The processor 110 may identify a patient's condition based on a result value acquired from the pre-trained neural network model 10. Specifically, the processor 110 may identify the presence or absence of a specific disease in a patient or the degree of possibility of a specific disease as the patient's condition. In particular, the processor 110 may classify the patient's condition into a plurality of condition grades based on a score indicating the degree of possibility of a specific disease acquired from the pre-trained neural network model 10. For example, when a score regarding the possibility of left ventricular systolic dysfunction is acquired from the pre-trained neural network model 10, the processor 110 may determine the condition of a new examinee as a normal grade when the score is less than a first value, determine the new examinee's condition as a stable grade when the score is equal to or greater than the first value and less than a second value, determine the new examinee's condition as a caution grade when the score is equal to or greater than the second value and less than a third value, determine the new examinee's condition as a severe grade when the score is equal to or greater than the third value and less than a fourth value, and determine the new examinee's condition as a critical grade when the score is equal to or greater than the fourth value. However, the present disclosure is not limited thereto, and the number of condition grades and a reference value of a score for distinguishing condition grades may be variously set.

On the other hand, the processor 110 may identify a patient's condition by combining scores for a plurality of diseases. For example, the processor 110 may identify a patient's condition by combining condition grades of a new examinee regarding left ventricular systolic dysfunction and arrhythmia.

According to an embodiment of the present disclosure, when a patient's condition is identified, the processor 110 may set a treatment order for a new examinee based on a time at which reception data of the new examinee was acquired and the new examinee's condition (S330). The processor 110 may set a treatment order for a new examinee to be later than the treatment orders of the other examinees waiting for treatment based on a reception time at which reception data of the new examinee was acquired. That is, the processor 110 may set a treatment order for a new examinee to be later than the treatment orders of the other examinees who visited and registered at a medical facility before the new examinee.

In this case, the processor 110 may set a treatment order for a new examinee by adjusting treatment orders of the other examinees who have already been registered and are waiting for treatment, based on the new examinee's condition. That is, when the processor 110 determines that the condition of an examinee is urgent, it may postpone the treatment orders of the other examinees who are waiting for treatment to be later than the treatment order of the new examinee, and may advance the treatment order of the new examinee.

In particular, when the processor 110 determines that the condition of a new examinee is urgent, it may advance the treatment order of the new examinee. To this end, the processor 110 may compare the new examinee's risk level with the risk levels of the other examinees waiting for treatment based on the new examinee's condition, and when it is determined that the new examinee's risk level is higher than those of the other examinees waiting for treatment, it may set the treatment order of the new examinee to be earlier than the treatment orders of the other examinees waiting for treatment.

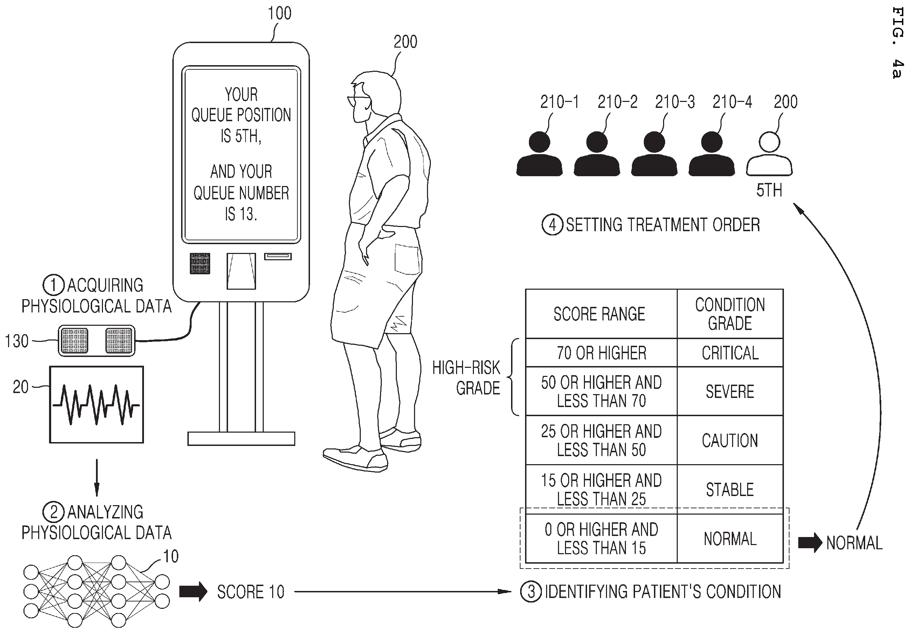

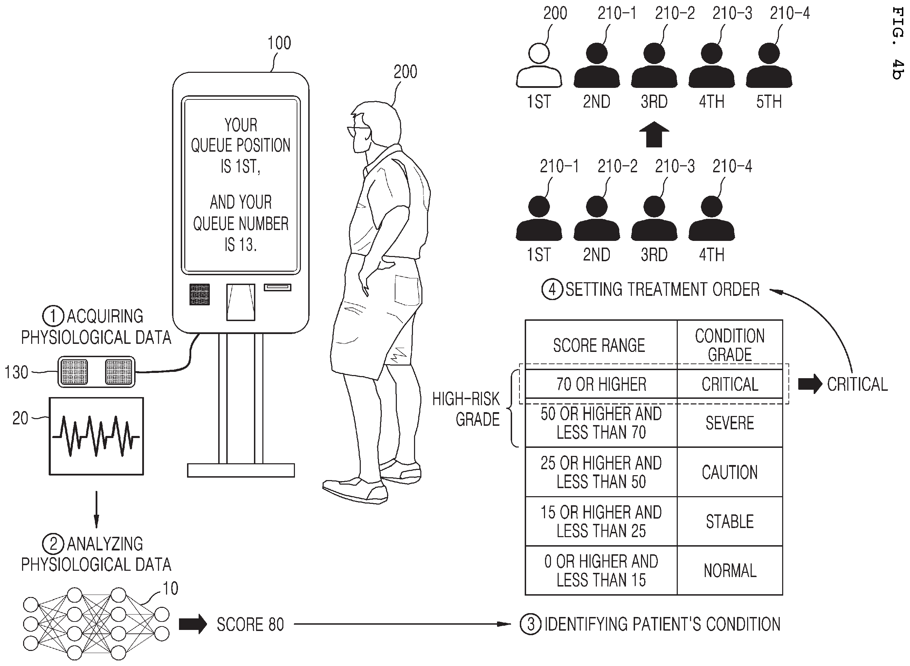

FIG. 4A and FIG. 4B are diagrams illustrating a method for setting a treatment order based on a patient's condition according to an embodiment of the present disclosure.

Referring to FIG. 4A, the processor 110 may input ECG data acquired from a new examinee 200 via the sensing unit 130 to a pre-trained neural network model 10 to acquire a score regarding the possibility of left ventricular systolic dysfunction of the new examinee 200. In this case, the processor 110 may identify the new examinee 200's condition based on the acquired score. The new examinee 200's condition is classified into a plurality of condition grades, and in particular, in the case of severe and critical grades among the plurality of condition grades, the processor 110 may identify that the new examinee 200's condition is risky. When the processor 110 determines that the new examinee 200's condition is normal (or stable, caution), it may set the treatment order of the new examinee 200 to the 5th position, which is later than the treatment orders of the other examinees 210-1 to 210-4 waiting for treatment.

On the other hand, referring to FIG. 4B, when the new examinee 200's condition is identified as a critical grade based on a score acquired based on examination data acquired via the sensing unit 130, the processor 110 may determine that the new examinee 200's condition is urgent, and may set the treatment order of the new examinee 200 to be earlier than the treatment orders of the four other examinees 210-1 to 210-4 waiting for treatment, i.e., to the 1st position.

In this case, according to an embodiment of the present disclosure, when there are a plurality of other examinees waiting for treatment, the processor 110 may identify another examinee with a condition grade lower than the condition grade of the new examinee 200 's risk level among the plurality of other examinees, and may set the treatment order of the new examinee 200 to be earlier than the treatment orders of the identified other examinee.

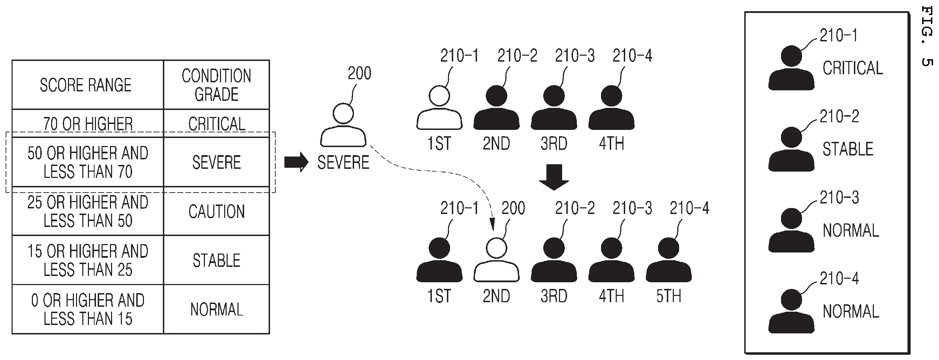

FIG. 5 is another diagram illustrating a method for setting a treatment order based on a patient's condition according to an embodiment of the present disclosure.

Referring to FIG. 5, when the new examinee 200's condition is identified as a severe grade based on the acquired score, the processor 110 may set the treatment order of the new examinee 200 to be earlier than the treatment orders of the other examinees 210-2 to 210-4 corresponding to condition grades lower than the severe grade among the four other examinees 210-1 to 210-4 waiting for treatment. That is, the processor 110 may set the treatment order of the new examinee 200 to be later than the treatment order of the other examinee 210-1 who is in the 1st position and in a critical grade state, but to be earlier than the treatment orders of the remaining other examinees 210-2 to 210-4, i.e., to the 2nd position.

On the other hand, when the condition grade of the new examinee 200 determined to be in a risky state matches the condition grade of another examinee waiting for treatment, the processor 110 may set the treatment order sequentially according to the time at which the reception data was acquired.

Referring back to FIG. 3, the processor 110 may provide queue number information corresponding to the treatment order of the new examinee 200 based on the set treatment order (S340). For example, the processor 110 may issue a queue number corresponding to the treatment order of the new examinee 200 and display the issued queue number via the display 140. In addition, the processor 110 may issue a queue number corresponding to the treatment order of the new examinee 200 and transmit the issued queue number to a terminal device (e.g., a smart watch) of the new examinee 200. Thereafter, the processor 110 may call the examinee to the examination room by displaying the queue number of the examinee whose treatment order is reached on the display 140 or outputting it via a speaker. In addition, it may also transmit information to the terminal device of the new examinee 200 indicating that their treatment order is reached.

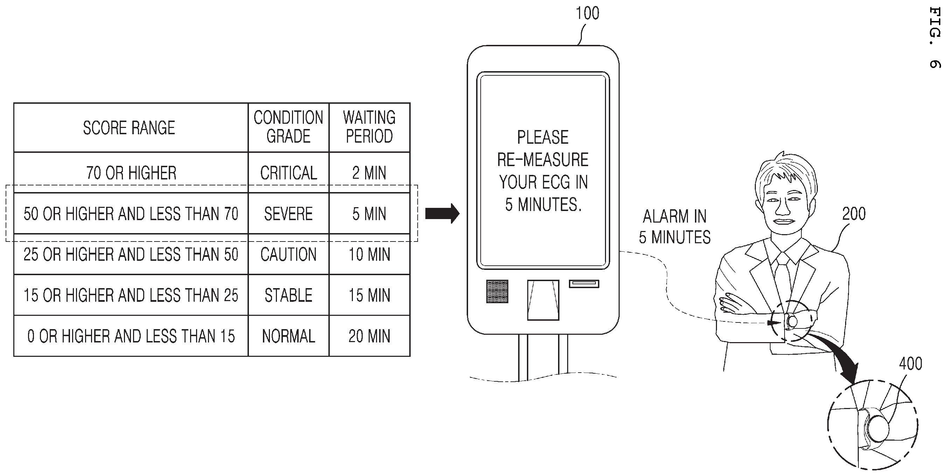

FIG. 6 is another diagram illustrating a method for setting a re-measurement period according to a patient's condition according to an embodiment of the present disclosure.

According to an embodiment of the present disclosure, when the condition of an examinee is classified into a plurality of condition grades, the processor 110 may set a re-measurement period for physiological data 20 corresponding to each condition grade. In this case, the processor 110 may identify a condition grade corresponding to the new examinee 200's condition, and may provide re-measurement time information for the physiological data 20 according to a re-measurement period corresponding to the condition grade of the new examinee 200 via the display 140. The same explanation as described above applies to the method of providing re-measurement time information. The higher the risk level according to the condition grade of the new examinee 200, the shorter the re-measurement period may be set.

For example, referring to FIG. 6, the processor 110 may preset a corresponding re-measurement period according to a condition grade of an examinee. Specifically, the processor 110 may set the re-measurement period to 20 minutes for a normal grade, 15 minutes for a stable grade, 10 minutes for a caution grade, 5 minutes for a severe grade, and 2 minutes for a critical grade. Then, the processor 110 may provide re-measurement time information calculated with the re-measurement period corresponding to the examinee's condition. For example, when the new examinee 200's condition is identified as a severe grade, the processor 110 may display re-measurement time information according to a 5-minute re-measurement period to the new examinee 200 on the display 140.

When the re-measurement time arrives, the processor 110 may display information requesting a re-measurement of the physiological data 20 of the new examinee 200 via the display 140, or transmit information requesting a re-measurement of the physiological data 20 to the terminal device 400 of the new examinee 200 via a communication interface. Specifically, the processor 110 may manage whether to perform re-measurement for a plurality of registered examinees including the new examinee 200. To this end, for an examinee whose re-measurement time has arrived, the processor 110 may display a message requesting a re-measurement of the physiological data 20 on the display 140 or transmit it to the terminal device 400 of the examinee via a communication interface. In addition, the processor 110 may output a voice message requesting a re-measurement of the physiological data 20 via a speaker of the computing device.

The processor 110 may determine whether each examinee has performed a re-measurement via the sensing unit 130 at a re-measurement time set for each examinee. After displaying or transmitting information requesting a re-measurement, when the processor 110 identifies that the new examinee 200 has not re-measured the physiological data 20 for a preset time, it may transmit information of the new examinee 200 to a terminal device of an administrator (e.g., an administration server). In particular, when the processor 110 determines that an examinee with a high risk level (e.g., when the condition grade of the examinee is severe or critical) has not performed a re-measurement for a set re-measurement time or within a preset time range based on the re-measurement time, it may notify an administrator's terminal device at a medical facility of the non-performance of re-measurement by the corresponding examinee via a communication interface, and may provide information about the corresponding examinee (e.g., name, gender based on reception data). In this case, the processor 110 may acquire location information of the corresponding examinee from a terminal device (e.g., a smartphone, a smart watch) of the corresponding examinee, and may provide the acquired location information to the administrator together to guide the administrator to quickly find the corresponding examinee.

On the other hand, according to an embodiment of the present disclosure, when physiological data 20 of a new examinee 200 is re-acquired via the sensing unit 130 upon re-measurement by the new examinee 200, the processor 110 may re-identify the examinee's condition based on the re-acquired physiological data 20, and may adjust the treatment order of the examinee based on the re-identified condition of the examinee. Specifically, when the condition grade of an examinee who has performed a re-measurement is changed and it is determined that the examinee's condition has a higher risk level than the other waiting examinees with the earlier treatment orders, the processor 110 may adjust the treatment order of the examinee whose condition has changed. The above description of the present disclosure can be identically applied to this, and thus a detailed description thereof is omitted.

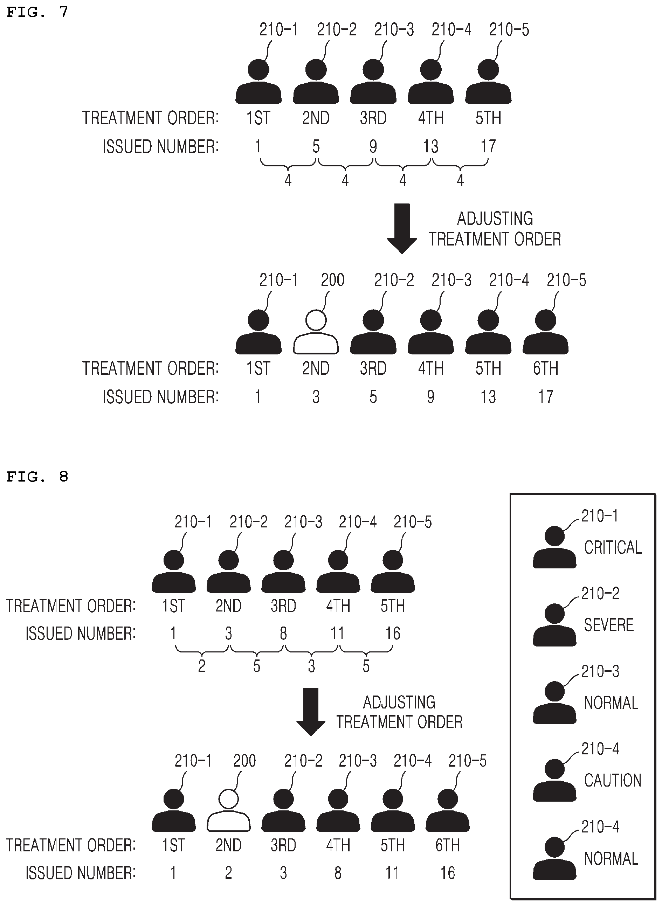

FIG. 7 is a diagram illustrating the issuance of queue numbers for examinees at a preset interval according to an embodiment of the present disclosure.

According to an embodiment of the present disclosure, queue numbers for the other examinees 210-2 to 210-5 who are waiting for treatment may be set and issued at a preset interval corresponding to the treatment orders of the other examinees waiting for treatment. Referring to FIG. 7, when the treatment orders for first to fifth examinees 210-` to 210-5 are set sequentially, queue numbers 1, 5, 9, 13, and 17 may be issued to the first to fifth examinees, respectively. That is, the interval set for the queue numbers may be 4. In this case, when the processor 110 sets a treatment order for a new examinee 200 to be earlier than the treatment orders of the other examinees waiting for treatment, it may maintain the queue numbers of the other examinees 210-2 to 210-5 whose treatment orders have been adjusted to be later, and may set and issue, for the new examinee 200, a queue number that falls within the preset interval and is earlier than the queue numbers of the other examinees whose treatment orders have been adjusted to be later.

That is, when the processor 110 determines that the condition of the sixth examinee 200 has a higher risk level than the conditions of the third to fifth examinees, it may set the treatment order for the sixth examinee 200 to be earlier than the treatment orders of the second to fifth examinees, but may set and issue the queue number as 3, which is one of the numbers existing in the interval between the first examinee and the second examinee. Accordingly, even if the treatment order of an examinee is changed, time and resources can be saved by not repeatedly issuing a queue number, and causing confusion to the examinee can also be prevented.

FIG. 8 is a diagram illustrating the issuance of a queue number for an examinee at an interval set according to a condition grade of the examinee according to an embodiment of the present disclosure.

On the other hand, the processor 110 may set the interval between issued queue numbers as an interval corresponding to the condition grade of another examinee waiting for treatment. In this case, the processor 110 may set a wider interval as the risk level according to the condition grade is lower, and may set a narrower interval as the risk level is higher. For example, referring to FIG. 8, the processor 110 may not set a preset interval for a critical grade, may set a preset interval to 2 for a severe grade, may set a preset interval to 3 for a caution grade, may set a preset interval to 4 for a stable grade, and may set a preset interval to 5 for a normal grade.

The processor 110 may set the interval by considering not only the condition grade of the examinee but also the congestion level within the medical facility. The congestion level may be identified by considering the number of patients who have visited the medical facility, the number of medical staff on duty, the presence or absence of an epidemic, and the like, and according to the congestion level, the processor 110 may correct the interval by assigning a weight to the interval set according to the condition grade of the examinee.

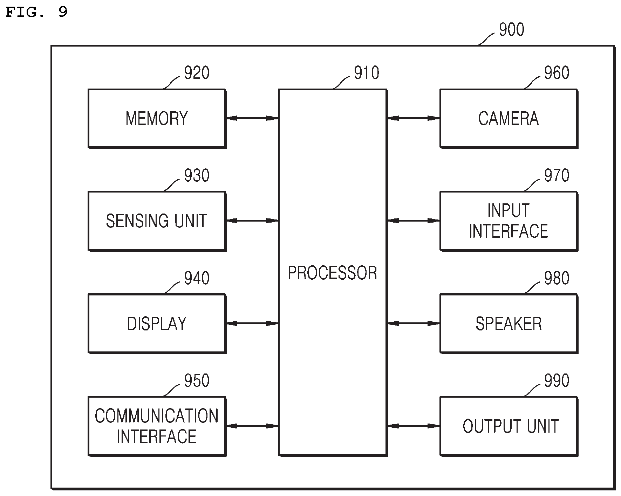

FIG. 9 is a configuration diagram of a computing device 900 according to another embodiment of the present disclosure.

Referring to FIG. 9, a computing device 900 according to another embodiment of the present disclosure may include one or more processors 910 (hereinafter, a processor 910), a memory 920, a sensing unit 930, a display 940, a communication interface 950, a camera 960, a user interface 970, a speaker 980, and an output unit 990. Among the components illustrated in FIG. 9, the processor 910, the memory 920, the sensing unit 930, and the display 940 correspond to the configurations of the processor 110, the memory 120, the sensing unit 130, and the display 140 of the computing device 100 illustrated in FIG. 2, and thus a detailed description thereof is omitted. In addition, the above description of the present disclosure based on FIGS. 1 to 8 may be identically applied to the computing device 900 illustrated in FIG. 9.

According to an embodiment of the present disclosure, the communication interface 950 according to an embodiment of the present disclosure may be understood as a component unit that transmits and receives data through any form of known wired/wireless communication system. For example, the communication interface 950 may perform data transmission and reception using a wired/wireless communication system such as a local area network (LAN), wideband code division multiple access (WCDMA), long term evolution (LTE), wireless broadband internet (WiBro), 5th generation mobile communication (5G), ultra wideband, ZigBee, radio frequency (RF) communication, wireless LAN, wireless fidelity (Wi-Fi), near field communication (NFC), or Bluetooth. The communication systems described above are only examples, and therefore, wired/wireless communication systems for data transmission and reception of the communication interface 950 may be variously applied in addition to the examples described above.

The communication interface 950 may receive data necessary for the processor 110 to perform operations via wired/wireless communication with any system or any client. In addition, the communication interface 950 may transmit data generated through the operations of the processor 110 via wired/wireless communication with any system or any client. For example, the communication interface 950 may receive patient data and medical data via communication with a database in a medical facility (and environment), a cloud server that performs tasks such as standardization of medical data, or a computing device. The communication interface 950 may transmit output data of the neural network model 10, intermediate data derived during the operation process of the processor 110, and processed data through communication with the aforementioned database, server, or computing device. For example, the processor 110 may acquire result data of the pre-trained neural network model 10 from an external computing device (e.g., an external server device) via the communication interface 950. In addition, the processor 110 may acquire physiological data 20 from an external computing device (e.g., a biosignal measurement device) via the communication interface 950, or may acquire training data for the neural network model 10.

The camera 960 captures an image of an object around a patient, including the patient, to acquire an image of the object. Specifically, the camera 960 may acquire an image of a patient. In this case, the processor 910 may analyze the acquired image of the patient to identify the patient and acquire pre-stored reception data, or to identify the patient's condition. To this end, the camera 960 may be implemented as an imaging device such as an imaging device with a CMOS structure (CIS, CMOS Image Sensor) or an imaging device with a CCD structure (Charge Coupled Device). However, the present disclosure is not limited thereto, and the camera 960 may be implemented as a camera module with various resolutions that can capture a subject. On the other hand, the camera 960 may be implemented as a depth camera (e.g., an IR depth camera), a stereo camera, or an RGB camera.

The user interface 970 is a component used by the computing device 100 to perform interaction with a patient, and may include at least one of a touch sensor, a motion sensor, a button, a jog dial, and a switch, but is not limited thereto. The processor 910 may receive patient information related to the patient's reception data, and the patient's biological information (e.g., occupation, age, gender) via the user interface 970.

The speaker 980 is a component that outputs various audio data on which various processing tasks such as decoding, amplification, and noise filtering have been performed by an audio processing unit (not shown). The speaker 980 may output various notification sounds or voice messages. According to an embodiment of the present disclosure, the processor 910 may convert an electrical signal into a user's (1) voice and output it via the speaker 980. For example, the speaker 980 may output a queue number of a patient whose treatment order is reached or a voice message calling a patient whose re-measurement time for physiological data 20 has come.

The output unit 990 performs outputting various information generated by the computing device 100. For example, the output unit 990 may include a printer device, and may output a paper on which a queue number of a patient (i.e., an examinee) is written using the printer device.

The various embodiments of the present disclosure described above may be combined with additional embodiments, and may be modified within the scope understandable by a person of ordinary skill in the art in light of the foregoing detailed description. The embodiments of the present disclosure are intended to be illustrative in all aspects and not restrictive. For example, each component described as a single unit may be implemented in a distributed manner, and likewise, components described as distributed may be implemented in a combined form. Therefore, all changes or modified forms derived from the meaning and scope of the claims of the present disclosure and their equivalent concepts are to be interpreted as being included in the scope of the present disclosure.

## Claims

1. A method for setting a treatment order based on a patient's condition, performed by a computing device including at least one processor, the method comprising:
when reception data of a new examinee is acquired, acquiring physiological data of the new examinee via a sensing unit;
analyzing the physiological data using a pre-trained neural network model to identify the new examinee's condition; and
setting a treatment order for the new examinee based on a time at which the reception data of the new examinee is acquired and the new examinee's condition,
wherein the setting of the treatment order comprises
setting the treatment order for the new examinee by adjusting, based on the new examinee's condition, the treatment orders of the other previously registered examinees who are waiting for treatment.

2. The method of claim 1, wherein
the setting of the treatment order for the new examinee comprises
setting the treatment order for the new examinee to be later than the treatment orders of the other examinees waiting for treatment, based on a reception time at which the reception data of the new examinee is acquired.

3. The method of claim 2, wherein
the setting of the treatment order for the new examinee by adjusting, based on the new examinee's condition, the treatment orders of the other previously registered examinees who are waiting for treatment comprises,
when it is identified, based on the new examinee's condition, that the new examinee's risk level is higher than the risk levels of the other examinees waiting for treatment, setting the treatment order for the new examinee to be earlier than the treatment orders of the other examinees waiting for treatment.

4. The method of claim 3, wherein:
the new examinee's risk level is classified into a plurality of condition grades; and
the setting of the treatment order for the new examinee to be earlier than the treatment orders of the other examinees waiting for treatment comprises,
when there are a plurality of other examinees waiting for treatment, identifying, from among the plurality of other examinees, an examinee having a condition grade lower than the condition grade of the new examinee's risk level, and setting the treatment order for the new examinee to be earlier than the treatment order of the identified examinee.

5. The method of claim 1,
wherein the new examinee's condition is classified into a plurality of condition grades, and a re-measurement period for the physiological data is set corresponding to each of the condition grades,
the method comprising
identifying a condition grade corresponding to the new examinee's condition, and providing re-measurement time information for the physiological data according to the re-measurement period corresponding to the new examinee's condition grade.

6. The method of claim 5, comprising,
when the re-measurement time is reached, displaying information requesting the re-measurement of the physiological data of the new examinee via a display of the computing device, or transmitting information requesting the re-measurement of the physiological data to a terminal device of the new examinee via a communication interface.

7. The method of claim 6, comprising,
when it is identified that, after displaying or transmitting the information requesting the re-measurement, the new examinee has not re-measured the physiological data for a preset time, transmitting information on the new examinee to a terminal device of an administrator.

8. The method of claim 6, comprising
re-identifying the examinee's condition based on the physiological data re-acquired from the new examinee via the sensing unit upon re-measurement by the new examinee, and adjusting the treatment order of the examinee based on the examinee's re-identified condition.

9. The method of claim 3,
wherein queue numbers of the other examinees waiting for treatment are set and issued at a preset interval corresponding to the treatment orders of the other examinees waiting for treatment,
the method comprising,
when the treatment order for the new examinee is set to be earlier than the treatment orders of the other examinees waiting for treatment, maintaining the queue numbers of the other examinees whose treatment orders have been adjusted to be later, and setting and issuing, for the new examinee, a queue number that falls within the preset interval and is earlier than the queue numbers of the other examinees whose treatment orders have been adjusted to be later.

10. The method of claim 9, wherein:
the condition of the other examinees waiting for treatment is classified into a plurality of condition grades; and
the preset interval is set to correspond to the condition grade of the other examinees waiting for treatment.

11. A computing device for setting a treatment order based on an emergency patient's condition, the computing device comprising:
a memory storing program code;
a sensing unit configured to acquire physiological data of an examinee; and
one or more processors configured to, when reception data of a new examinee is acquired, acquire physiological data of the examinee via the sensing unit, analyze the physiological data using a pre-trained neural network model to identify the new examinee's condition, and set a treatment order for the new examinee based on a time at which the reception data of the new examinee is acquired and the new examinee's condition,
wherein the one or more processors are configured to set the treatment order for the new examinee by adjusting, based on the examinee's condition, the treatment orders of the other previously registered examinees who are waiting for treatment.
